# EUROPEAN PATENT APPLICATION

(11) **EP 2 338 557 A1**
(43) Date of publication of application: **29.06.2011**
(21) Application number: 09180578.8
(22) Date of filing: 23.12.2009
(51) Int. Cl.: A61M 37/00

(54) **Soluble microneedle**

(71) Applicant: DEBIOTECH S.A., 1004 Lausanne (CH)
(72) Inventor: Mefti, Selma, 1004, Lausanne (CH); Cachemaille, Astrid, 1004, Lausanne (CH); Piveteau, Laurent-Dominique, 1004, Lausanne (CH)
(74) Representative: Grosfillier, Philippe

(57) **Abstract**

Microneedle comprising a distal part substantially made of a soluble material, characterized by the fact that said distal part comprises a stiffening structure.

## Description

### Field of the invention

The present invention concerns the administration of drugs with microneedles. More specifically, it concerns microneedles comprising a part which dissolves by hydrolysis once micro-needles have penetrated the skin.

### Background art

Soluble microneedles are mostly constituted by biodegradable polymers. They can be made from maltose, polylactic acid (PLA), carboxymethylcellulose (CMC), hyaluronic acid or silicone. The polymer is mixed with a drug, the whole mixture being then made in the form of microneedles. Once into the skin, microneedles dissolve more or less rapidly by hydrolysis, thus releasing the drug.

Different forms and compositions of microneedles are described in the following patent publications: US 2009/0182306 A1, US 2009/0035446 A1 and WO 2008/130587 A2.

State-of-the-art microneedles may be manufactured according to different processes, in particular:

### - Centrifuge casting

This method is described by Lee et al in Patent US2009/0182306 A1.
First a female master structure is fabricated thanks to microelectronics facilities. The mold can be produced using a SU-8 photolithography to create conical (circular cross-section) or pyramidal (square cross-section) microneedle. By this method the microneedles have a base of 300 um and a length about 600 um to 800 um. The tips have a radius of 25 um. The mold can also be made of silicon by etching process. A male mold in PDMS for example is created using the first mold. For a better release from the mold, the PDMS mold can be sputtered by gold. PDMS is chosen due to its ability to conformally coat microneedles, its good adhesion and for its easy separation of the microneedle for the mold and finally because of its price.
Then the microneedle matrix is prepared. The polymer is dissolved in deionized water, the water is evaporated until obtaining the desired concentration and which gives a viscous mixture. The concentration is given by measuring solution mass before and after the evaporation and the viscosity by using a Couette viscometer. The polymer is heated until 60-70ºC to be concentra ted under vacuum or an ambient pressure.
If necessary a drug can be added by hand mixing to solubilize or suspend the active agent in the final hydrogel.
Few micrograms of the hydrogel is put on the PDMS mold in a conical centrifuge tube and centrifuged at 45° during 2 hours. The cen trifugation allows filling microneedle cavities and drying the mixture.
This technique enables several layers of polymer containing or not some drug by repeating the last step several times.

### - Pillar patterning

This method is described by Jung et al in Patent US2008/0108959 A1.
This method is described with PLA, CMC and maltose.
First the CMC is dissolved in water in order to obtain the desired concentration. The obtained solution is then coated on a flat glass panel with the desired thickness and put into contact with pillars (here a frame with 2x2 pillars with a diameter of 200 um). The CMC is dried to increase adhesion between the pillars and the polymer. To form the polymer microneedles the coated CMC is drawn at 30um/s during 60 seconds.
Finally the needle are dried during 5 minutes and separated from the frame. This step could be by cutting or by increasing the speed.
Microneedle 1800 um long and with a 5um upper diameter can be obtained.
A similar process is followed for PLA and maltose microneedle.

### - Compression

This method is described by Takao Tomonon the Patent US2008/0208134 A1.
A first master mold designed with microneedles' shape is fabricated. Using this master a recessed mold is created.
This replicated recessed mold is pressed against a biodegradable polymer matrix which is heated. After the cooling down the matrix is separated form the replicated mold. Finally the polymer microneedle film is cut out to desired dimensions. The polymer can also be chitin or chitosan.

State-of-the-art microneedles however present several disadvantages.
First, according to the chosen material, the mechanical resistance is not always sufficient to insert them into the skin. For example microneedles can bend or break before their insertion.
Furthermore, it is known that with microneedles of relative short length, e.g. 600 µm, the module of Young which is necessary to penetrate the skin has to be of the order of some GPa (cf. " Dissolving Microneedle for Transdermal Drug Delivery ", Jeong Woo Lee, Jung-Hwan Park and mark R. Prausnitz, Biomaterials, May 2008.). Such constraints thus limit the choice of the polymer, the microneedles shape as well as their aspect factor.
In addition, the manufacturing processes according to the prior art do not allow to obtain tips made of polymer as thin as those of the microneedles made of silicon. This inconvenience has for consequence a relative weak depth of penetration of microneedles, ie to a third, even a quarter of the total length of microneedles. Finally, the needles being of the order of hundred of microns, the amount of drug scattered in microneedles is relatively weak.

### Summary of the invention

The present invention offers a solution to the previously cited problems.

It concerns a microneedle comprising a part constituted of a soluble material, the said part containing a stiffening structure, also named skeleton in the present application

The stiffening structure can be located inside, outside or simultaneously inside and outside of said part.
It can be made of metal (e.g, steel or titanium), plastic, silicon, ceramic or polymer (e.g. soluble, biodegradable or swelling).

It can also be constituted of a soluble or stable material, having some rigidity, but with dissolution rate which is less than the dissolution rate of said soluble material.

Any suitable shape can be used with the microneedle according to the invention, e.g. conical, pyramidal, a cylinder topped with a cone (here the sharp tip), or a parallelepiped mounted by a pyramid (here the sharp tip), or a more complex shape.
The microneedle may also be a blade.

The invention also encompasses devices including one or several such microneedles.

In one embodiment the stiffening structure of the microneedle is located inside said distal part. The inner structure may be a stalk, a set of stalks, and a plate flat or curved.

In another embodiment the stiffening structure of the microneedle is located on the external face of said distal part. In the case of a conical microneedle, the structure can be a partial conical envelope. In the case of a pyramidal microneedle, the structure can be one or more sides of the pyramid. For cylindrical microneedle the structure may be a part a cylinder including or not a part of the sharp tip. With a parallelepiped microneedle the structure may be one or more sides of the parallelepiped including or not one or more sides of the sharp tip.
The external skeleton can even be more complex and may be silicon microneedle using cleanroom facilities. The microneedles may be obtained by dry or wet etching. Examples of process are described by Stemme in the patent application WO03/015860 and by Nanopass in the patent WO01/66065A1.

In another possible embodiment the stiffening structure of the microneedle may be located inside and on the external face of said distal part. The stiffening structure may be a combination of above-mentioned shapes.

In this invention the soluble material of the microneedle can be a biodegradable polymer. Suitable soluble polymers may include but not limited to Polylactic Acide (PLA), Polyglycolic Acid (PGA), poly(lactic-co-glycolic acid) (PLGA), Cellulose, Sodium Carboxymethyl Cellulose (SCMC), Hydroxyethyl cellulose (HEC), Hydroxypropyl cellulose (HPC), hydroxypropyl methylcellulose (HPMC), Amylopectin (AMP), Hyaluronic acid, silicone, Polyvinylpyrolidone (PVP), Polyvinyl alcohol (PVA), Poly(vinylpyrrolidone-co-methacrylic acid) (PVA-MAA), Polyhydroxyethylmethacrylate (pHMEA), Polyethlene glycol (PEG), Polyethylen oxide (PEO), chrondroitin sulfate, dextrin, dextran, maltodextrin, chitin, chitosan, mono and polysaccharides, galactose, maltose.
This list is not exhaustive.

In another embodiment the microneedle contains one, two or more polymers with the same dissolution rate. These above-mentioned polymers may be distributed in different ways in the microneedle : horizontally, vertically, with a defined angle, as envelopes, or a combination of these said distributions .
These polymers may contain one or more active ingredients.

In another embodiment the microneedle contains one, two or more polymers with the same dissolution rate including one active ingredient with different concentration.
In a preferred embodiment the soluble material is constituted of several biodegradable polymers having each a specific dissolution rate, containing one active ingredient at different concentrations.
In another preferred embodiment the soluble material is constituted of several biodegradable polymers having each a specific dissolution rate, containing several active ingredients.

In all the above-cited embodiments the said distal part of the microneedle contains an active ingredient. The active ingredient may be a drug, an enzyme, a vaccine with or without its adjuvant, a diagnostic agent, a vitamin, or a protein.

Furthermore the microneedle may include a proximal part formed by a non soluble material. This proximal part ensures to have the complete "active part" of microneedle under the skin.

The distal part may be a metal, a ceramic, a polymer, a soluble polymer, a biodegradable, a swelling polymer, a plastic, or silicon.

The presence of the stiffening structure allows to produce microneedles of very diversified forms, with a sharper tip and of improved mechanical resistance. With such a configuration a facilitated penetration and an increased depth of penetration can be achieved.

### Detailed description of the invention

The invention will be better understood below with examples illustrated by the following figures :
Figure 1 illustrates some soluble microneedles according to the prior art.
Figure 2 illustrates several configurations of soluble microneedles according to the invention with an inner or external stiffening structure.
Figure 3 is a 3D representation of configuration 5 of figure 2.
Figure 4 illustrates several configurations of soluble microneedle according to the invention with an inner or external stiffening structure and a base at the bottom part.
Figure 5 illustrates several configurations of soluble microneedle according to the invention with an inner or external stiffening structure and a fast dissolving part.
Figure 6 illustrates several types of soluble microneedles according to the invention for bolus or continuous delivery
Figure 7 is a picture of a soluble microneedle with an external skeleton (viewed by fluorescence microscopy)

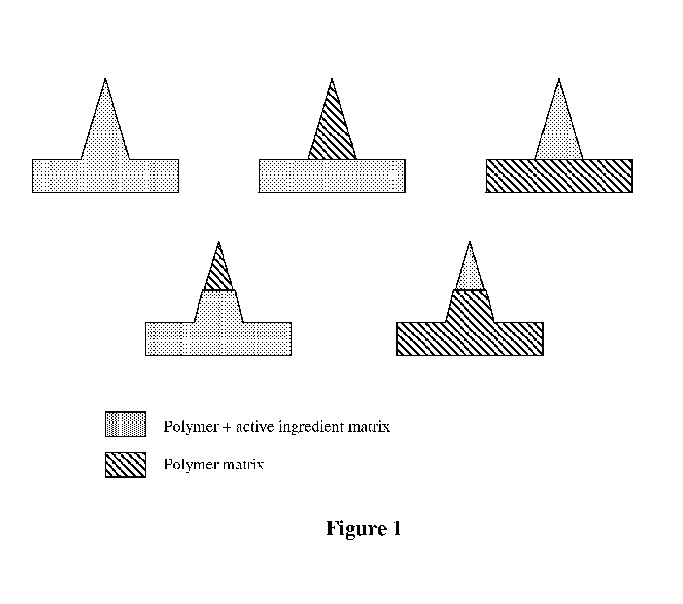

The microneedles illustrated in figure 1 are shown in cross-section. Their base can be square or round. In most of cases the drug is concentrated near the tip of the microneedle as shown on illustration 6 or is only concentrated in the microneedle itself (see illustration 3).
Here the microneedles do not have any stalk and the microneedles dissolve completely under the skin. When dissolution occurs pathways under the skin are created and allow the drug contained in the soluble part of the microneedle to flow under the skin. Lee et al described the case in US 2009/0182306 A1 application with a swelling polymer.

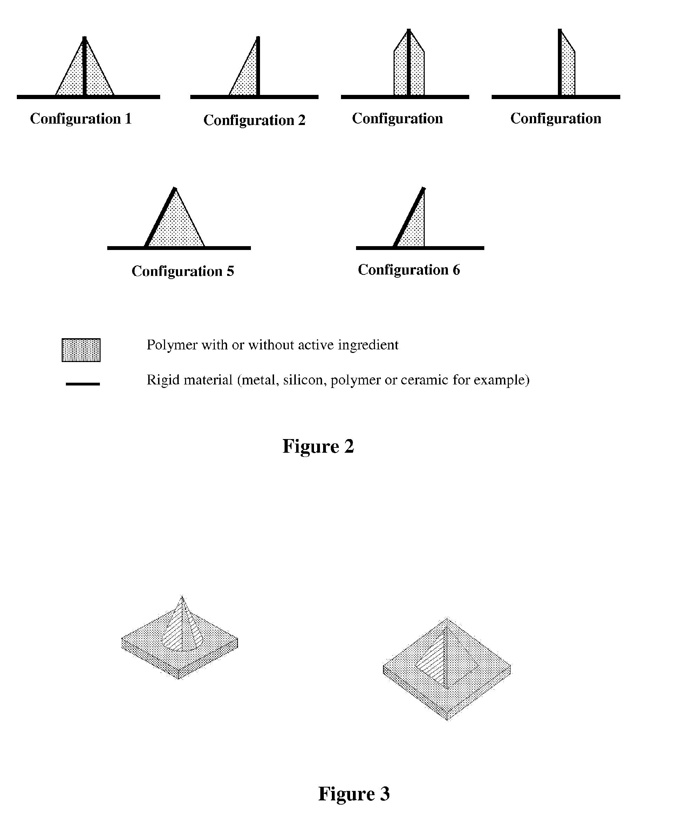

The microneedles as illustrated in figure 2 are shown in cross-section. They contain a stiffening structure represented by a vertical or oblique dark line, the clear zone of microneedles being formed by one or several soluble materials which contains one or several drugs.
The several polymers can be arranged in different ways: horizontally, vertically with an angle or even as an envelope.

The stiffening structure has the shape of a stalk or of a set of stalks. It can also have the shape of plate, flat or curved. As example, it can have the shape of a partial conical envelope, one or several sides of a pyramid.
The configuration and/or the composition of microneedles can vary according to the envisaged application, punctual administration of the drug (bolus) or progressive (continuous delivery).
For the injection of a bolus, a polymer with fast dissolution is preferably chosen while we shall opt for a polymer with slow dissolution for a continuous delivery. This structure can be constituted by a rigid material as e.g. a metal, silicon, plastic, ceramic or a polymer (biodegradable, soluble, swelling or not).
This structure allows to pierce the skin and avoid the bending of the microneedle. The processes described in the state of the art do not allow to get a sharp tip, so the microneedles have to be designed (aspect ratio and choice of the polymer) to resist to the insertion without bending. Here the sharp structure pierces the skin, thus any polymer and aspect ratio can be chosen.

Configurations No 1 and 3 in figure 2 show internal skeletons. The other configurations (2, 4, 5, 6) contain external skeletons.

The bottom part of a microneedle may have any shape, e.g. round or square (see Figure 3).

In certain cases, during the insertion, microneedles do not penetrate completely into the skin. So, a drug which would be towards the bottom part of microneedles could not be used.

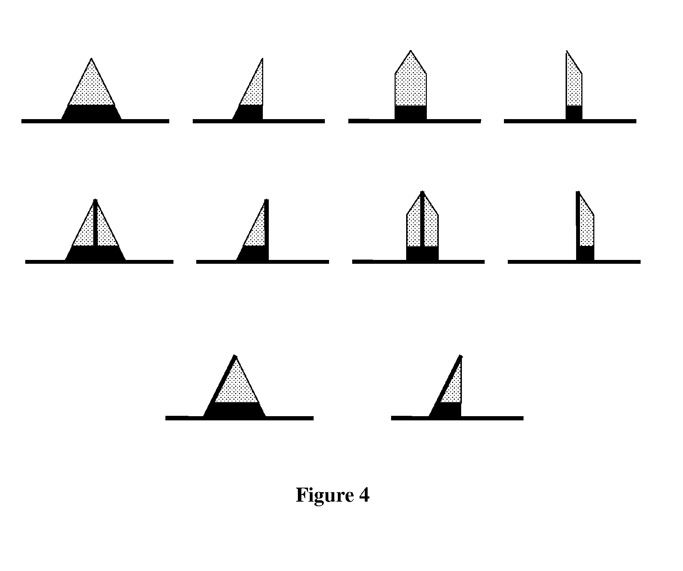

To avoid this absence of efficiency and increase the rigidity of the set, the microneedles can contain a base at the level of their bottom part (see Figure 4). This base can be constituted by a rigid material as e.g. a metal, silicon, ceramic, plastic or a polymer.
If the mechanical resistance of the soluble polymer is not sufficient to pierce the skin during the injection, the stiffening structure can also be placed or exclusively placed at the level of the tip of microneedles. All microneedles of Figure 4 contain a stiffening structure also placed at the level of the tip.

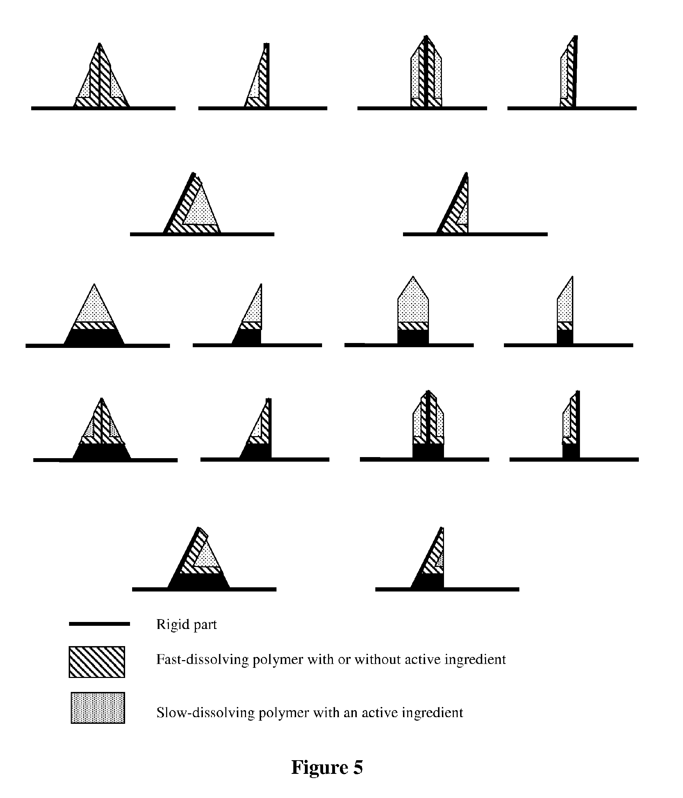

It is also possible (see Figure 5) to add a material with fast dissolution (in dark gray in Figure 5) between the fixed part (base) and the soluble part containing the drug. This transition part allows to remove quickly the fixed part of the microneedles without having to wait that the soluble portion releases all the drug. In a preferred embodiment the microneedle includes a proximal part formed by a non soluble material. The presence of this proximal part ensures to have the entire soluble (active) part under the skin.

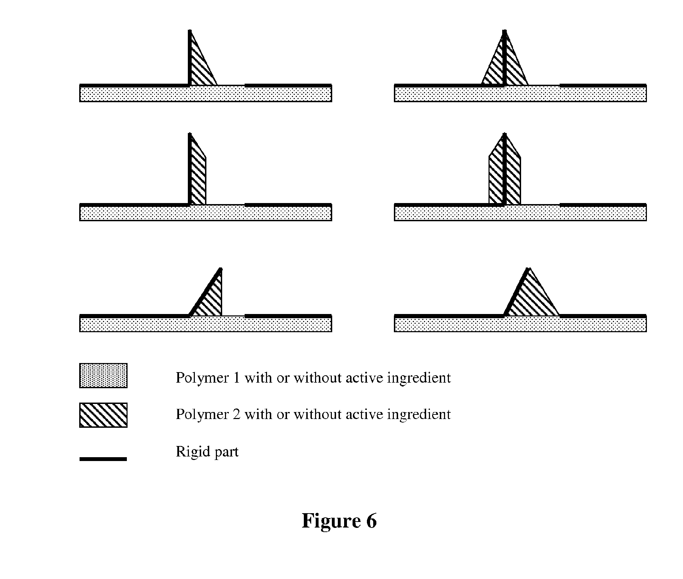

Figure 6 illustrates another embodiment of the invention wherein the stiffening structure also extends until the substrate on which is based the microneedles. This last one can also be made of a soluble material and contain a drug. For example a laser cutting of the stiffening structure allows to arrange openings in the substrate, thus offering passages for the drug contained in the substrate.
The opening on the substrate allows the diffusion of the drug contained in the substrate into the skin. The microneedle pierces the skin and its dissolution enables pathways into the skin. These pathways which are open during several hours allow the dissolution of the drug contained into the substrate.

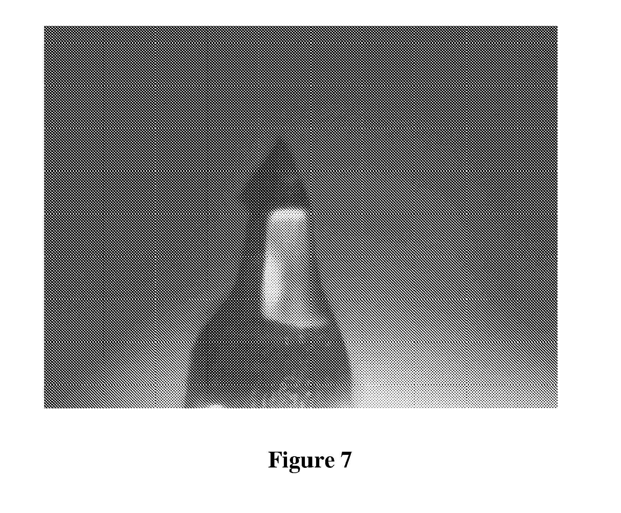

Figure 7 illustrates a soluble microneedle with an external skeleton which may be obtained by the following process : First silicon microneedles are obtained using photolithography techniques and dry etching in clean room facilities. The chips are diced and stuck or bonded to a connector and mounted to a syringe. Then few milligrams of agar gel (a soluble polymer) and ICG (Indocyanine Green used for medical diagnostics for determining cardiac output, hepatic function, liver blood flow, and also for ophthalmic angiography) are mixed together with some water. The whole mixture is boiled during 5 minutes. A green gel is obtained. The syringe and the microneedles are filled with the still warm mixture. Here the microneedle is used as a mold and no other mold is required. Finally the microneedles are put in a cold environment to solidify the gel.

A device may include only one polymer. This polymer can contain one active ingredient in the microneedle itself and the same active ingredient in the backing layer but at a different concentration.
Alternatively the device may still include only polymer, but with two different active ingredients, one in the microneedle itself and the other in the baking layer.
In a preferred embodiment a device with two different soluble polymers can be used. The first polymer contained in the microneedle can be fast-dissolving polymer for a bolus injection (e.g CMC, chitin, chitosan or maltose) with some drug. The second polymer can be a slow-dissolving polymer for a continuous delivery (e.g PLA or PLGA) and contain the same or another active ingredient. The difference between the self-dissolution rates can be low or higher depending on the desired application.
The several polymers can be arranged in different ways: horizontally, vertically with an angle or even as an envelope.
These embodiments are described for two polymers but it can also be described for three or more polymers. For example, several polymers or active ingredients may be contained in the microneedle as well as several polymers or active ingredients can be included in the baking part.

The above-mentioned embodiments solve the issue of the low amount of drug scattered in microneedles.

## Claims

1. Microneedle comprising a distal part substantially made of a soluble material, **characterized by** the fact that said distal part comprises a stiffening structure.

2. Microneedle according to claim 1 wherein the stiffening structure is located inside said distal part.

3. Microneedle according to claim 1 wherein the stiffening structure is located on the external face of said distal part.

4. Microneedle according to claim 1 wherein the stiffening structure is located inside and on the external face of said distal part.

5. Microneedle according to any of the previous claims wherein the soluble material is a biodegradable polymer.

6. Microneedle according to claim 5 wherein the soluble material is constituted of several biodegradable polymers having each a specific dissolution rate.

7. Microneedle according to any of the previous claims wherein said distal part contains an active ingredient.

8. Microneedle according to any of the previous claims wherein the stiffening structure is a metal, silicon, a ceramic or a non-biodegradable polymer.

9. Microneedle according to any of the previous claims wherein the complete microneedle is substantially made of a soluble material.

10. Microneedle according to any of the previous claims 1 to 8 having a
proximal part formed by a non soluble material.

11. Assembly comprising a substrate on which are located one or several
microneedles as defined in any of the previous claims.
